# EUROPEAN PATENT APPLICATION

(11) **EP 1 749 479 A1**
(43) Date of publication of application: **07.02.2007**
(21) Application number: 05425575.7
(22) Date of filing: 02.08.2005
(51) Int. Cl.: A61B 17/02, A61B 17/28

(54) **Retractor instrument**

(71) Applicant: Gandini, Marco, 10040 Almese (Torino) (IT)
(72) Inventor: Gandini, Marco, 10040 Almese (Torino) (IT)
(74) Representative: Freyria Fava, Cristina

(57) **Abstract**

The present invention relates to a retraction instrument (1, 100) comprising an elongated tubular body (3, 103) to which are associated a rod (2, 102, 122) and at least one deployable/orientable formation (5, 105, 155) in the distal position with regard to the principal body (3, 103) and to the rod (2, 102, 122) where the formation (5, 105, 155) can move from a retracted position of alignment with the principal body (3, 103) towards at least one deployed position in which the formation (5, 105, 155) extends inclined with regard to the principal body (3, 103).

## Description

The present invention concerns instruments to retract objects, preferably organs or tissues in general and in particular the abdominal wall. The present invention also concerns instruments that are also predisposed or appropriate for incision, separation, suturing and gripping objects, in particular organs, tissues or foreign bodies within an organism.

### STATE OF THE ART

Various mechanisms have been proposed for the realisation of retraction or gripping instruments, in particular in the surgical field.

Retraction instruments for use in laparoscopy generally comprise a body and one or more mobile appendices. They are inserted into pre-existing or especially-created body cavities through small incisions.

These instruments are constituted in such a fashion that they can be manipulated and activated from outside the cavity. Once the distal part of the instrument has been inserted into the cavity, this is activated so as to achieve movement of the mobile appendices which take on positions such that they can exercise force to contain or retract organs, tissues or masses.

Instruments for the retraction of the abdominal walls generally comprise pieces in a single part or in multiple parts, in some cases articulated one with another, that are introduced within the abdominal cavity through a small incision and then disposed or manoeuvred so as to achieve a more extensive contact area. They are subsequently connected to other instruments outside the abdominal cavity that exercise traction. This traction exercised on the abdominal wall causes a more extensive space to be created within the abdominal cavity, necessary to carry out laparoscopic surgery.

With regard to gripping instruments, whether laparoscopic or traditional, they generally comprise a handgrip (ring-type, pistol-grip, etc.), a body and two or more jaws of varying length and shape. Those used for laparoscopy are of reduced diameter, but the dimensions of the jaws are also reduced. In particular, the length of the mobile parts of the surgical staplers are reduced since, given the length of the body it is difficult to exercise an adequate force to obtain good closure and application of the metal staples.

Systems for anchoring trocars are chiefly comprised of inflatable hollow structures (balloons) which can be inflated through a tube and a valve. Alternatively, trocars can also be anchored with sutures and their extraction made more difficult through knurling of the surfaces.

### DESCRIPTION OF THE INVENTION

The purpose of the present invention is to provide new instruments for the retraction of objects, in particular organs and tissues, that present advantages in terms of their use by the surgical team.

The present invention has an analogous purpose in relation to instruments for retraction that are also predisposed or appropriate for incision, separation, suturing and gripping objects, in particular organs, tissues or foreign bodies.

In accordance with the present invention, this purpose is achieved thanks to solutions that are described specifically in the claims below. The claims form an integral part of the technical instruction provided here in relation to the invention.

Reference will henceforth be made, as a simple and non-restrictive example, to the case of instruments for the retraction of human or animal organs or tissues.

The present invention concerns instruments for the retraction of organs or tissues that can be used with advantage also in the absence of pneumoperitoneum (in the case of a retractor applied to the abdominal wall) that enable continual variation of the inclination of the portion of the instrument whose purpose is to exercise retraction of the organ or tissue and that can be used both in normal and in obese subjects.

Instruments for the retraction of organs or tissues as per the present invention also provide the advantage that they can be inserted into hollow organs and, for example, that they enable the passage of a tube for enteral feeding.

Where instruments as per the present invention are utilised as anchorage systems for a trocar, they present the big advantage of exercising greater force and providing greater stability than the anchorage instruments currently in use.

Furthermore, in the case of instruments for the retraction of organs or tissues as per the present invention being predisposed for the incision, separation, or gripping of organs, tissues or foreign bodies, the instruments for gripping and/or incision can reach and operate at different angles from that of the principal part of the instrument itself.

The instruments for the retraction of organs or tissues, for the incision, separation and gripping of organs, tissues or foreign bodies, present the further advantage given by the possibility of operating with more degrees of freedom, that is of moving one part with respect to another part of any instrument by a variable number of degrees, in a stable and repeatable fashion.

A further advantage of the gripping instruments as per the present invention is given by the possibility offered to the surgeon to exercise a certain force including at a relatively large distance from the handgrip.

The invention further concerns instruments able to bear inside them optical fibres. This has the advantage, given the possibility to articulate the instrument, of being able to visualise parts hidden behind other organs.

The present invention concerns instruments that can bear surgical staplers at their distal extremity. The advantage is that the articulation mechanism of the present invention enables the application of considerable force on the distal part including with considerable length of the body. This is particularly useful for laparoscopic surgical staplers.

The invention will now be described in detail, as a simple non-restrictive example, with reference to the attached figures, in which:
-- figure 1 illustrates a section of a first embodiment of a retraction instrument as per the present invention;
-- figure 2 illustrates a front view of a second embodiment of the retraction instrument as per the present invention;
-- figure 3 illustrates a section along the line II-II of figure 2;
-- figure 4 illustrates an enlargement of the detail indicated with the arrow III in figure 2;
-- figure 5 is a section along the line V-V in figure 4;
-- figures 6-10 illustrate in diagram form the positions that can be taken on by the parts of the instrument as per the present invention, indicated by arrows I and III in figures 1 and 3;
-- figure 11 illustrates a third embodiment of the detail indicated by arrow I in figure 1;
-- figure 12 illustrates a second position of the detail in figure 11;
-- figure 13 illustrates a section of a third embodiment of the retraction instrument as per the present invention;
-- figure 14 is an elevation illustrating the retraction instrument in figure 13 in a second position.

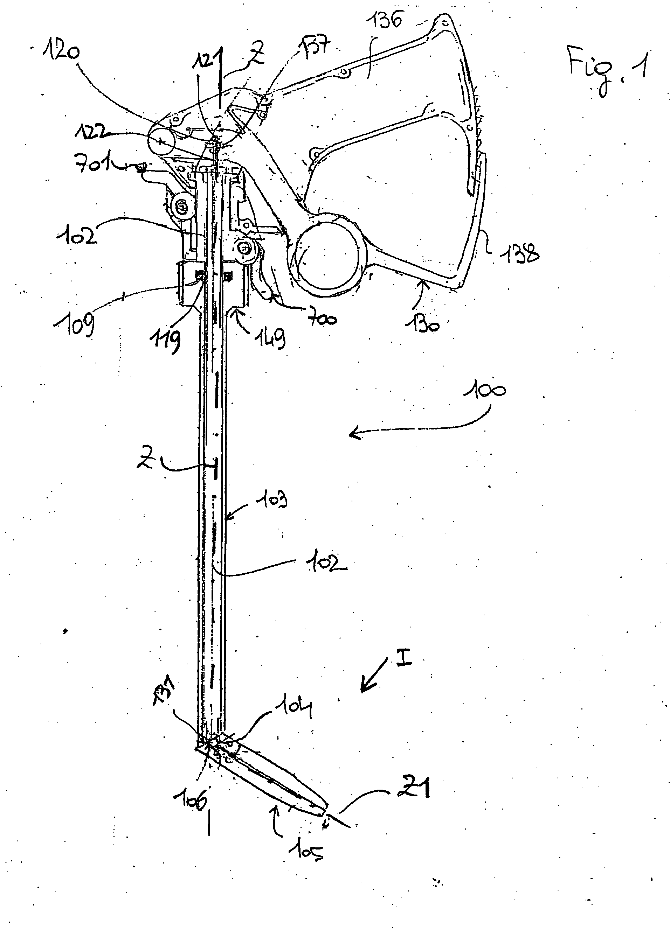

A first embodiment of an instrument for the retraction of organs or tissues in general is illustrated in figure 1.

The proximal extremity of the retraction instrument -- indicated overall with the reference 100 in figure 1 -- is destined to be manipulated by the surgeon and presents a handgrip, 130, whereas the distal extremity is destined to exercise the functions proper to the instrument.

The instrument comprises a tubular body 103 a rod 102 situated inside the tubular body 103 and mounted such that it can rotate around the axis Z of the body 103. The distal extremity of the body 103 is cut obliquely forming a first skew surface 131 inclined with regard to the longitudinal axis Z.

The rod 102 presents a circular, elliptical or polygonal section and, at its distal extremity, it is connected through a first joint 104 to an orientable formation, 105.

The rod 102 may be hollow so as to enable the passage of tubes, air, gas, liquids, endoscopic optical devices or of a control device, 120, to activate instruments coupled to the orientable formation 105 such as for an example forceps, scissors or surgical staplers.

The proximal extremity of the body 103 is solidly linked to a knurled wheel 149. Rotating the wheel 149 a rotary movement around the axis Z is impressed onto the body 103. This movement may be halted at any point by a first blocking system 700 capable of blocking the wheel 149.

Furthermore, rotation of the rod 102 the wheel 149 and in consequence the body 103 may be independent with regard to the handgrip 130. Said rotation movement may the halted at any point through a second blocking system 701.

The proximal parts of the rod 102 and of the body 103 are linked through a linking device 109 constituted for example of a wing-nut structure integral with the rod 102 able to co-operate with a corresponding housing 119 in the wheel 149.

The control 120 to operate the instruments linked to the orientable formation 105 in a embodiment that is at present preferred, consists of a cable 122 of various materials, flexible, connected to the portion 138 of the handgrip 130. The portion 138 is provided with a hollow structure 137 that houses within it the terminal part 121 of the cable 122 that preferably presents a spherical shape. Operation of the handgrip 130 -- which in figure 1 presents a trigger conformation -- produces a translation movement along the axis Z of the cable 122 and thus operates the instrument connected to the first orientable formation, 105.

The orientable formation 105 in a first embodiment comprises a cylindrical structure, optionally hollow, elongated along the longitudinal axis Z1 and of dimensions similar to those of the body 103. The proximal extremity of the formation 105 presents an oblique cut that forms a second skew surface 106 inclined with respect to the longitudinal axis Z1 which co-operates with the first skew surface 131 of the body 103. At its distal extremity, the formation 105 -- as illustrated in figure 1 - bears forceps.

The formation 105 is integral in rotation with the rod 102 and may move between:
-- an introduction position in which it is aligned with the body 103;
-- a deployed working position in which the orientable formation 105 is inclined with respect to the body 103.

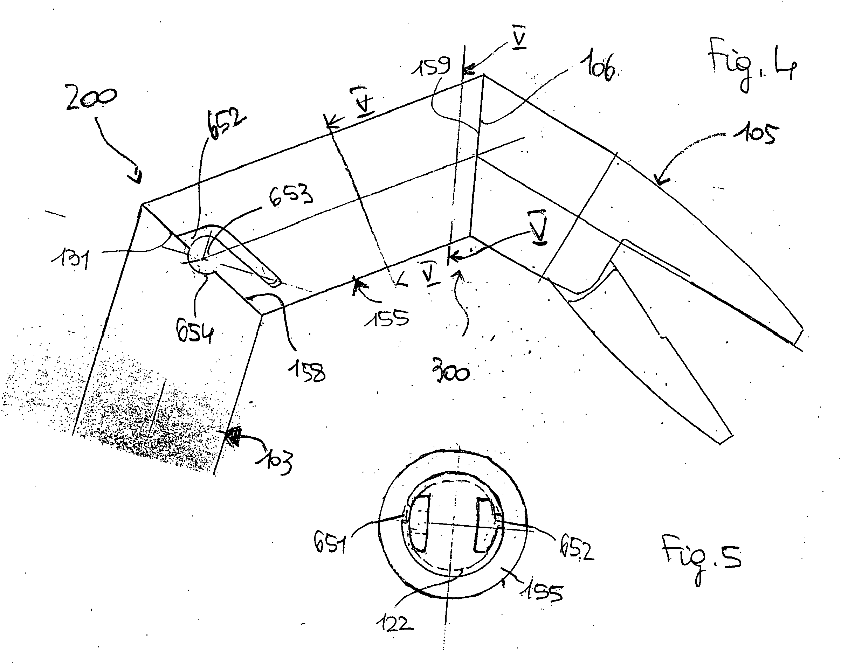

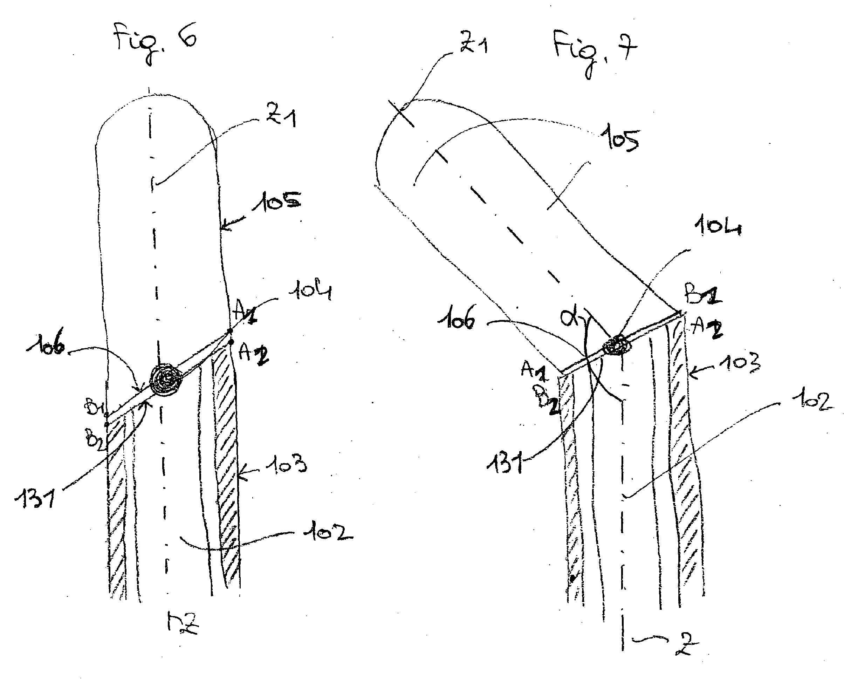

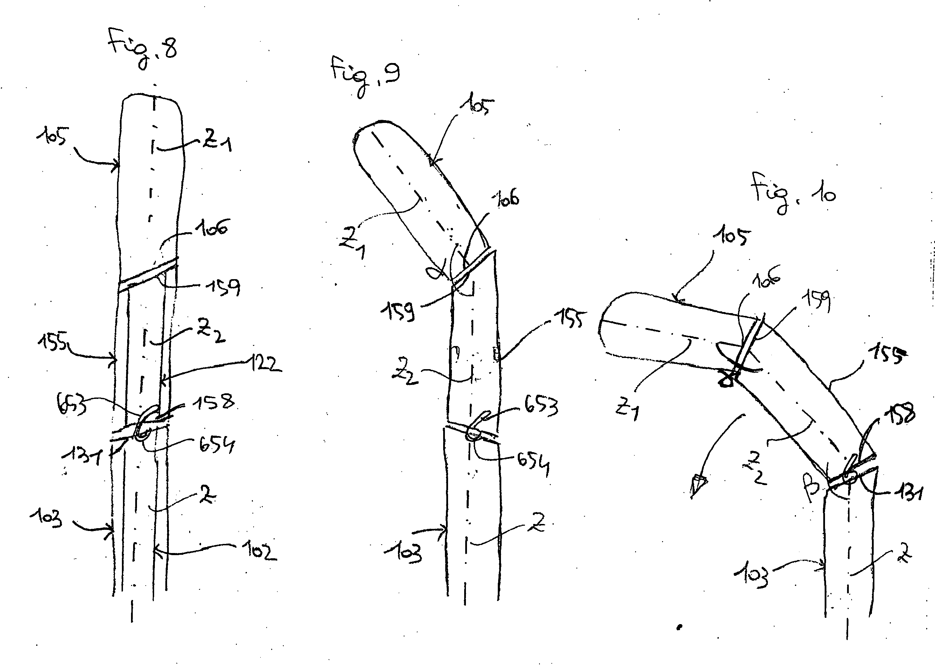

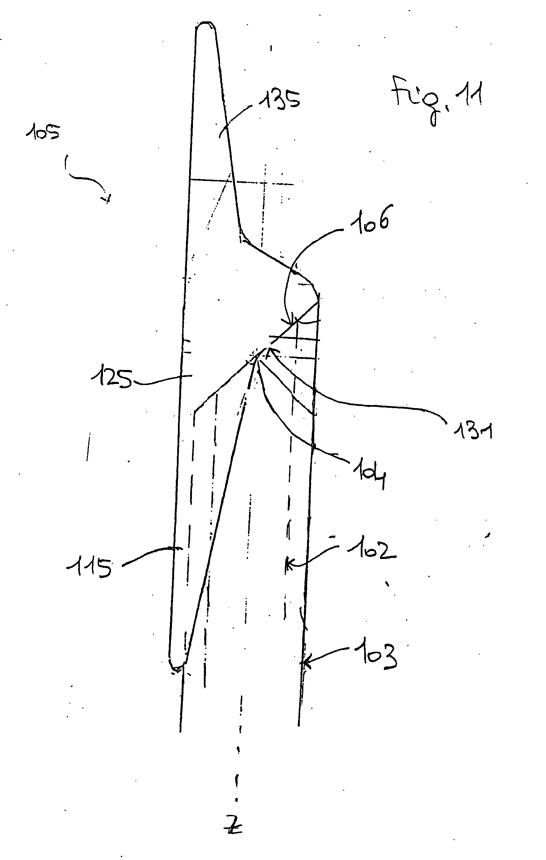

With regard to figures 6 and 7, the rotary movement of the body 103 -- controlled via the wheel 149 -- brings the formation 105 from a position of alignment with the axis Z to a second deployed position in which the two axes Z and Z1 form an angle α. The second skew surface 106 co-operates in a sliding bearing interaction with the first skew surface 131 whereby rotation of the body 103 by effect of the interaction between the two skew surfaces 106 and 131 orients the first orientable formation 105 into the deployed working position. More specifically, to reach the maximum deployed position illustrated in figure 7 a rotary movement of 180° of the body 103 with regard to the formation 105 - integral with rod 102 -- brings points A2 and B2 of the first skew surface 131 initially facing points A1 and B1 of the second skew surface, 106 (when the formation 105 is aligned with the body 103) to be exchanged: in the position of maximum deployment, point B2 faces point A1 and point A2 faces point B1.

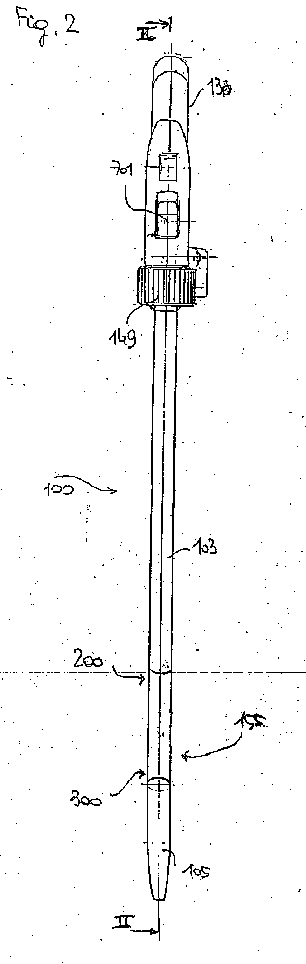

The orientable formation 105 may present various conformations. As an example, some conformations of the orientable formation 105 will now be described; they are illustrated in diagram form in figures 2, 3 and 4.

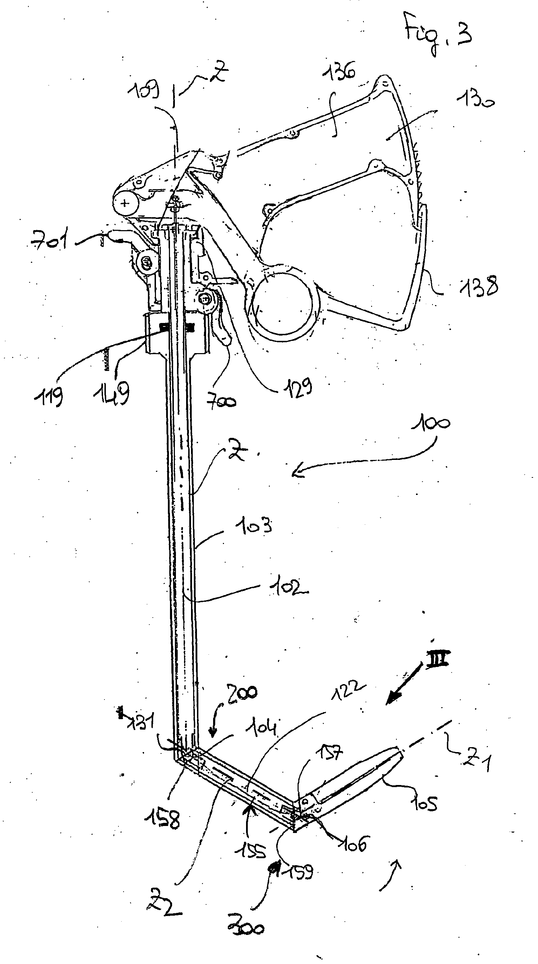

With reference to figures 2 and 3, a variant of the orientable formation 105 provides for the presence of an intermediate orientable formation 155. The intermediate orientable formation 155 is situated between the first orientable formation 105 and the body 103.

The intermediate formation 155 presents a hollow cylindrical structure with longitudinal axis Z2 and dimensions substantially similar to those of the body 103. The proximal extremity 200 of the intermediate formation 155 presents an oblique cut which forms a third skew surface 158 inclined with respect to the longitudinal axis Z2 and co-operating in a sliding bearing interaction with the first skew surface 131 of the body 103. The distal extremity 300 of the intermediate formation 155 presents a further oblique cut which forms a fourth skew surface 159 inclined with respect to the longitudinal axis z2 and co-operating in a sliding bearing interaction with the second skew surface 106 of the first orientable formation 105.

Inside the intermediate formation 155 there is an intermediate rod 122 which is connected through a first joint 104 to the rod 102 and through a second joint 157 to the formation 105.

With reference to figure 4, the intermediate formation 155 presents at its proximal extremity 200 the first locking devices 653 which engage with the corresponding second locking devices 654 present on the body 103 such that the intermediate formation 155 and the body 103 are made to move integrally. The first locking devices 653 are substantially comprised of a convex structure, preferably of a semi-spherical or semi-cylindrical shape, the second locking devices 654 are substantially comprised of the concave structure preferably of a semi-spherical or semi-cylindrical shape, capable of coupling with the first locking devices 653. There are two each of the first and second locking devices 653 and 654 in diametrically opposed positions.

The formation 155 presents, in correspondence with the first locking devices 653 a slot 652 which enables the first locking devices 653 to come out of the concave structure of the second locking devices 654 thus disengaging the movement of the intermediate formation 155 from the body 103 thus enabling its free rotation around its axis Z2 and thereby enabling movement of the formation 155 with respect to the body, 103.

The intermediate formation 155 also presents in the central section on its internal surface, a first tooth 651 susceptible of co-operating with a second tooth 652 present on the external surface of the second rod 122 as illustrated in figure 5.

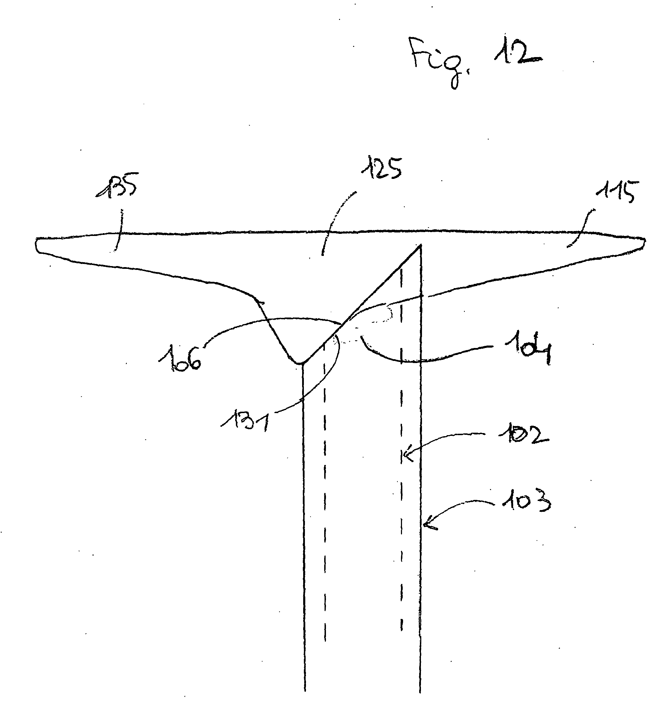

The movement of the two deployable formations 105 and 155 is illustrated making reference to figures 8, 9 and 10.

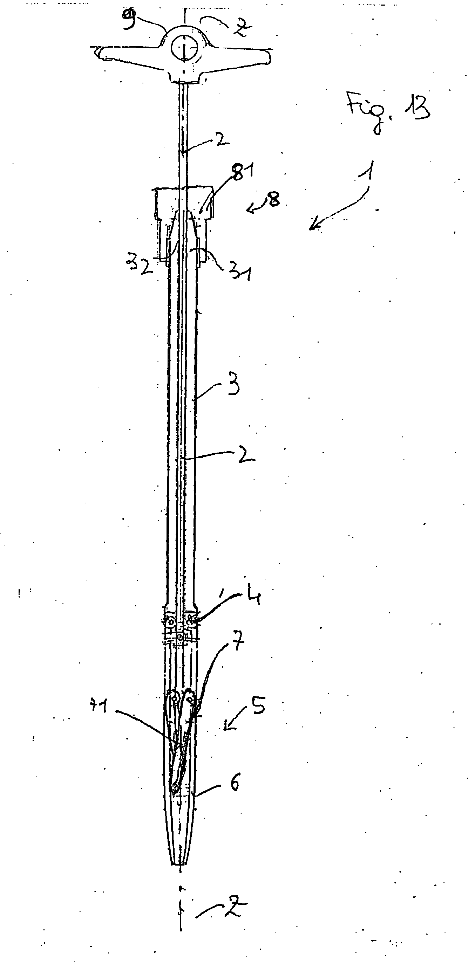

The rotary movement in a first direction of the body 103 integral with the formation 155 thanks to the first and second locking devices 653 and 654 -- by exploiting the sliding bearing interaction of the fourth skew surface 159 of the intermediate formation 155 and of the second skew surface 106 of the first formation 105 - enables the instrument to be disposed in the configuration illustrated in figure 9, that is deployment of the sole formation 105 where the axis Z1 of the first formation 105 forms an angle α with the longitudinal axis Z2 of the intermediate formation 155 aligned with the axis Z of the body 103.

Once this configuration has been reached, movement in the same direction of the formation 155 is blocked by the co-operation of the first tooth 651 of the intermediate formation 155 with the second tooth 652 of the second rod 122. Movement of the body 103 and hence of the formation 155 in the opposite direction would bring the first formation 105 back to the configuration illustrated in figure 8.

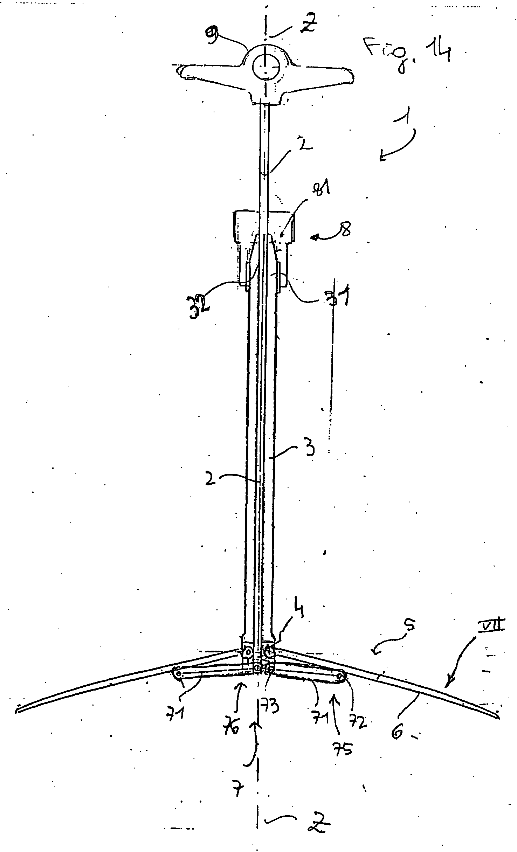

To reach the configuration illustrated in figure 10, in which the longitudinal axis Z2 of the intermediate formation 155 forms an angle β with the axis Z of the body 103 it suffices to proceed to a further rotation of the body 103 in the first direction.

More specifically, by impressing a rotation on the wheel 149 and in consequence on the body 103 the locking devices 653 and 654 are disengaged. By effect of the sliding interaction between the first skew surface 131 and the third skew surface 158 respectively of the body 103 and of the intermediate formation 155 the intermediate formation 155 is oriented in the deployed working position illustrated in figure 10. The position reached may be maintained by blocking the movement of the body 103 by activating the first blocking system 700.

The position of maximum deployment of the intermediate formation 155 can be reached after two rotations of 180° of the wheel 149 so as to enable the locking devices 653 and 654 to engage with the locking devices diametrically opposed to the initial ones.

Figure 11 illustrates a variant of the orientable formation 105. In this conformation the formation 105 is comprised of three portions 115, 125, 135. The proximal portion 115 and the distal portion 135 which present variable length and elliptical, polygonal, semicircular or other section, form a single monolithic body with the central portion 125.

The central portion 125 is connected to the rod, 102 via a joint 104 and presents a second skew surface 106 inclined with respect to the axis Z of the body 103 co-operating in a sliding bearing interaction with the first skew surface 131 of the body 103 as a function of the rotation of the body 103. The formation 105 thus passes from the conformation illustrated in figure 11 to that illustrated in figure 12, with the two portions, the distal portion 135 and the proximal portion 115 in an almost orthogonal position with respect to the axis Z of the body 103.

A second embodiment of the retraction instrument for organs or tissues in general as per the present invention is illustrated in figures 13 and 14.

The proximal extremity of the retraction instrument -- indicated overall with reference number 1 -- is destined to be manipulated by the operator, whereas the distal extremity is destined to exercise the functions proper to the instrument.

The retraction instrument 1 comprises a tubular body 3 with associated to it an operating rod 2 which can slide axially in the body 3 a first joint 4 between the body 3 and at least a first deployable formation 5.

The deployable formations 5 may be present in various numbers, may be of different lengths and different shapes.

The deployable formation 5 may adopt:
-- a retracted position aligned with the operating rod 2 (illustrated in figure 13); and
-- a deployed position, in which the formation extends inclined with regard to the axis Z of the body 3 as a function of the movement of the operating rod 2 with relation to the body 3 (illustrated in figure 14).

Connected to the operating rod 2 there is an operating mechanism 7 (for example bend type or pantograph type) which transforms the axial movement of the operating rod 2 inside the body 3 into a movement of the deployable formation 5 between a retracted position and a deployed position.

The operating mechanism 7 as illustrated in figures 13 and 14, comprises a series of bars 71 in number equal to the number of elements constituting the deployable formation 5 having an elongated shape and connected at the distal extremity 75 via second joints 72 to the individual elements 6 constituting the deployable formation 5. The bars 71 are connected at their proximal extremity 76 via third joints 73, to the operating rod 2.

The operating mechanism 7 enables the arms 6 of the deployable formation 5 to be deployed in function of movement of the rod 2 along the axis Z: starting from the conformation illustrated in figure 13, retracting the rod 2 upwards causes the bars 71 and thus the arms 6 to splay achieving the deployed configuration illustrated in figure 14.

A variant of the retraction instrument 1 as per the present invention, provides for the use of two concentric operating rods to which are articulated, through their respective operating mechanisms, respective deployable formations 5 comprising a number of arms 6 articulated one with another.

The section of the arms 6 is preferably elliptical but may also be circular, polygonal or other.

The body 3 holds a blocking system 8 of the instrument 1 to avoid any movement of the operating rod 2 with respect to the body 3 when the deployable formations 5 are in the deployed position inside the patient's body.

A type of block 8 consists of a ring-nut 81 which engages on the body 3 via a screw-thread. In this variant, the body 3 bears at its proximal extremity two diametrically opposed incisions/cuts of adequate length that generate two hemi-valves 31 and 32. Rotating the ring-nut 81 clockwise causes constriction of the two hemi-valves 31 and 32, of the body 3 on the rod 2 blocking their movement.

A variant of the blocking system 8 (not illustrated) may consist of a type two lever with fulcrum on the body 3 resistance being given by a spring placed about half way along the lever and that unites it to the body 3 and enables application of a manual force at the proximal extremity of said lever. In the upper third of said lever, a tooth is situated that, with the instrument open, is housed in a special hole in the rod.

At the proximal extremity of the operating rod 2 there is a coupling system 9 (for example ring-type or screw-type) which enables it to be connected to raising systems.

The rod 2 may have a hollow interior enabling the passage of air, liquids or other instruments such as optical probes or catheters for enteric feeding.

A further variant of the retraction system 1 as per the present invention, provides for the use of an operating rod 2 which can slide parallel to the body 3 and is linked to it via appropriate coupling systems (for example dovetail-type).

In the particular case of a retraction instrument predisposed for gripping organs or tissues, the proximal extremities of the operating rod 2 and of the body 3 are included in an operating mechanism of the pistol type or similar. In this case, the deployable formations 5 may be connected to a cloth or material in sheet form appropriate to facilitate gripping fragments. The tissue retraction instrument 1 may also be used for anchoring a cannula, in which case the operating rod 2 of the instrument 1 constitutes the cannula itself.

The above-described instruments may be made in various forms or sizes to enable their use in traditional surgery, laparoscopic surgery or endoscopy in general.

With regard to the materials from which the instruments are made, as per the present invention, such materials may be plastic, resin, metal or alloy or again, biocompatible materials whether resorbable or not.

### Fields of application

The applications in the surgical fields of the above-described instruments are immediately clear to experts in the field. However, for purely descriptive and non-limiting purposes they may be described thus:
-- as retractor of the abdominal wall for gasless laparoscopy, that is without pneumoperitoneum (in particular the practical forms as described in figures 11 to 14);
-- as retractor of a hollow organ, after introduction into the organ itself, for example to favour the introduction of cannulas, optical systems, washing systems, catheters for parenteral nutrition, etc. (in particulars the practical forms illustrated in figures 11 and 14);
-- as endoscopic retractor of organs, tissues (figures 1 to 4);
-- as articulated endoscopic instrument to support forceps, scissors, surgical staplers or other instruments (figures 1 to 4);
-- as retraction systems in general including in industrial applications.

Naturally, the construction details and practical forms may be widely varied with regard to what is described and illustrated here without thereby falling outside the sphere of protection of the present invention, as defined in the attached claims.

## Claims

1. Retraction instrument (100) comprising:
- a tubular body (103) elongated in the direction of its longitudinal axis (Z) presenting in the distal position a first skew surface (131) inclined with respect to said axis (Z);
- at least one rod (102, 122); and
- at least one orientable formation (105,155) elongated in the direction of its longitudinal axis (Z1, Z2) in the distal position with regard to said body (103) and to said rod (102, 122) and each presenting at least one skew surface (106, 138, 159) inclined with respect to said axis (Z), where said formation (105, 155) can move from a retracted position of alignment with said axis (Z) towards at least one deployed position in which said formation (105, 155) is inclined with respect to said axis (Z), and where said at least one formation (105, 155) is coupled to said body (103) in such a way that the rotary movement in one direction of said body (103) produces a deployment movement of said at least one formation (105, 155) and movement in the opposite direction of said body (103) produces a recall movement of said at least one formation (105, 155), where said rotary movement involves the movement in sliding bearing interaction of at least two skew surfaces (131, 106, 158, 159).

2. Retraction instrument according to claim 1, **characterised in that** said first orientable formation (105) presents a cylindrical structure, optionally hollow, having similar dimensions to those of said body (103) and bears in the proximal position a second skew surface (106) co-operating with said first skew surface (131) of said body (103).

3. Retraction instrument according to claim 1, **characterised in that** said intermediate orientable formation (155) presents a hollow cylindrical structure having dimensions similar to those of said body (103) and bears a third skew surface (158) in the proximal position (200) co-operating with said first skew surface (131) of said body (103) and a fourth skew surface (159) in the distal position (300) co-operating with said second skew surface (106) of said first formation (105).

4. Retraction instrument according to claim 1, **characterised in that** said rod (102, 122) is mounted in such a fashion that it can rotate around said axis (Z) within said body (103) or within said intermediate orientable formation (155).

5. Retraction instrument according to claim 1, **characterised in that** said rod (102) is connected at its distal extremity to said at least one deployable formation (105, 155) by means of a first joint (104).

6. Retraction instrument according to the previous claim, **characterised in that** said intermediate rod (122) is connected at its proximal extremity to said rod (102) via a joint (104) and at its proximal extremity it is connected to said first formation (105) via a second joint (157).

7. Retraction instrument according to claim 1, **characterised in that** said rod (102, 122) is hollow.

8. Retraction instrument according to any of the previous claims, **characterised in that** said body (103) is integrally connected to a wheel (149) that controls the mechanism of rotation around said axis (Z) of said body (103).

9. Retraction instrument according to any of the previous claims, **characterised in that** said instrument is provided with a blocking system (700) of the movement of said body (103).

10. Retraction instrument according to any of the previous claims, **characterised in that** said instrument is provided with a handgrip (130) at its proximal extremity.

11. Retraction instrument according to any of the previous claims, **characterised in that** said intermediate formation (155) presents at its proximal extremity (200) first locking devices (653) engaging with second locking devices (654) present on said body (103).

12. Retraction instrument according to the previous claim, **characterised in that** there are two each of said first and second locking devices (653, 654) and in diametrically opposed positions.

13. Retraction instrument according to claim 11, **characterised in that** said intermediate formation (155) presents in the central section, on the inner surface a first tooth (651) that can co-operate with her second tooth (652) present on the external surface, in the central section of said intermediate rod (122).

14. Retraction instrument according to any of the previous claims, **characterised in that** said first formation (105) is comprised of three portions (115, 125, 135) where said central portion (125) is connected to said rod (102) by means of a joint (104) and is provided, in the proximal position, with a second skew surface (106) co-operating with said first skew surface (131) of said body (103).

15. Retraction instrument according to the previous claim, **characterised in that** said three portions (115, 125, 135) of said deployable formation (105) are formed in a single monolithic body.

16. Retraction instrument according to claim 1, **characterised in that** said first formation (105) is coupled to instruments useful for retraction or to surgical staplers.

17. Retraction instrument (1) comprising:
-- a tubular body (3) elongated in the direction of its longitudinal axis (Z);
-- at least one operating rod (2); and
-- at least one deployable formation (5) in the distal position with respect to said body (3) and to said rod (2);
-- at least one bar (71) associated to each of said at least one deployable formation (5), said at least one bar (71) connected at its proximal extremity to said at least one operating rod (2);
in which said formation (5) can move from a retracted position of alignment with said axis (Z) towards at least one deployed position in which said formation (5) is inclined with respect to said axis (Z), and where said at least one formation (5) is coupled to said operating rod (2) in such a fashion that translation movement along said axis (Z) of said at least one operating rod (2) determines -- thanks to co-operation in the movement of said at least one bar (71) -- a deployment movement of said at least one formation (5) and movement in the opposite direction of said at least one operating rod (2) determines a recall movement of said at least one formation (5).

18. Retraction instrument according to claim 17, **characterised in that** said operating rod (2) is mounted such that it can slide along the axis (Z) inside said body (3).

19. Retraction instrument according to either claim 17 or claim 18, **characterised in that** said body (3) is connected at its distal extremity to said at least one deployable formation (5) by means of a first joint (4).

20. Retraction instrument according to claims 17, 18 or 19, **characterised in that** said at least one bar (71) is connected at its proximal extremity through third joints (73) to said operating rod (2).

21. Retraction instrument according to any of claims 17 to 20, **characterised in that** said at least one bar (71) is connected at its distal extremity through second joints (72) to each of said at least one deployable formation (5).

22. Retraction instrument according to any of the claims 17 to 21, **characterised in that** said at least one deployable formation (5) is constituted of at least one arm (6).

23. Retraction instrument according to any of the claims 17 to 22, **characterised in that** said body (3) is provided at its proximal extremity with two hemi-valves (31, 32) that embrace said rod (2).

24. Retraction instrument according to any of the claims 17 to 22, **characterised in that** said body (3) is provided with a blocking element (8) of the axial movement of said rod (2).

25. Retraction instrument according to the previous claim, **characterised in that** said blocking element (8) comprises a ring-nut (81) whose rotation around said axis (Z) determines the constriction of said two hemi-valves (31, 32) on said rod (2), blocking its movement.

26. Retraction instrument according to claim 17, **characterised in that** said operating rod (2) is externally coupled to said body (3) via suitable coupling systems.

27. Retraction instrument according to any of the claims 17 to 26, **characterised in that** said rod (2) is hollow.
